(19) **Europäisches Patentamt / European Patent Office / Office européen des brevets**

(11) **EP 2 330 410 A2**

(12) **EUROPEAN PATENT APPLICATION**
published in accordance with Art. 153(4) EPC

(43) Date of publication:
**08.06.2011 Bulletin 2011/23**

(21) Application number: **09817999.7**

(22) Date of filing: **30.09.2009**

(51) Int Cl.:
*G01N 27/407* (2006.01)    *G01N 27/417* (2006.01)
*G01N 27/414* (2006.01)    *H01L 29/786* (2006.01)
*H01L 29/06* (2006.01)    *G01N 27/26* (2006.01)
*G01N 27/12* (2006.01)

(86) International application number:
**PCT/KR2009/005615**

(87) International publication number:
**WO 2010/038989 (08.04.2010 Gazette 2010/14)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **30.09.2008 KR 20080096088**
**24.10.2008 KR 20080104809**
**30.09.2009 KR 20090093057**

(71) Applicants:
• **Iljin Copper Foil Co., Ltd.**
**Iksan-si**
**Jeollabuk-do 570-998 (KR)**
• **Cios Inc.**
**Yuseong-gu, Daejeon-city 305-701 (KR)**

(72) Inventors:
• **PARK, Jin Su**
**Daejeon-city 302-781 (KR)**
• **YOON, Byung Young**
**Yeongi-gun**
**Chungcheongnam-Do 339-761 (KR)**
• **PARK, Jung Won**
**Daejeon-city 305-759 (KR)**
• **CHO, Jung Hwan**
**Yeongi-gun**
**Chungcheongnam-Do 339-761 (KR)**
• **KIM, Sang Beom**
**Yeongi-gun**
**Chungcheongnam-Do 339-824 (KR)**

(74) Representative: **Kling, Simone**
**Lavoix Munich**
**Bayerstrasse 85a**
**80335 München (DE)**

(54) **NITROGEN-OXIDE GAS SENSOR**

(57)    The present invention provides a nitrogen-oxide gas sensor that is able to measure nitric oxide and nitrogen dioxide at the same time and ensure measurement accuracy and long stability. The present invention relates to the nitrogen-oxide gas sensor that includes: an oxide ion conductive solid electrolyte; a primary film made of a metal oxide which contacts the solid electrolyte; a secondary film made of a metal oxide that contacts with the solid electrolyte and is separated from the first film; a power source that applies electric power to the primary and secondary films by electrically connecting a primary node to the primary film and a secondary node to the secondary film; a tertiary film made of a metal oxide that contacts the solid electrolyte, wherein the tertiary film and the primary film are connected to the power source in parallel; and a measurement unit that measures the electric potential difference between the primary and secondary nodes.

EP 2 330 410 A2

FIG. 1

**Description**

TECHNICAL FIELD

The present invention relates to a nitrogen-oxide gas sensor, and more particularly, to a nitrogen-oxide gas sensor that is able to increase sensing accuracy and ensure long stability.

BACKGROUND ART

A nitrogen-oxide gas is indicated as NOx since it includes a nitric oxide (NO), a nitrogen dioxide (NO$_2$), or a nitrous oxide (N$_2$O). In this regard, the nitric oxide and the nitrogen dioxide occupy most of the nitrogen-oxide gas, and act as atmospheric pollution sources. Thus, concentrations of the nitric oxide and the nitrogen dioxide are measured to suitably control emission amounts.

A conventional method of using equilibrium potential may be used to measure concentration of a nitrogen-oxide gas. In this regard, an electrochemical cell is formed by using a nitrate in a solid state as a sensing electrode in a solid electrolyte, and forming a precious metal electrode to uniformly maintain ion activity in the solid electrolyte, and the concentration of the nitrogen-oxide gas is measured by using an electromotive force of the electrochemical cell. However, a melting point of the sensing electrode is low, and thus, such a conventional method cannot be applied to a high temperature gas.

Alternatively, the concentration of the nitrogen-oxide gas may be measured by using a current type sensor. In this regard, a nitrogen dioxide is converted to a nitric oxide by using an oxygen pumping cell, and a current generated by oxygen ions obtained by decomposing the nitric oxide is measured to determine the concentration of the nitrogen-oxide gas. However, such a method is structurally limited since the oxygen pumping cell is used, and it is difficult to measure a total amount of the nitrogen-oxide gas since the measured current largely changes according to temperature and is very small when the concentration is below or equal to hundreds of ppm.

Alternatively, the concentration of the nitrogen-oxide gas may be measured by using mixed dislocation. In this regard, a sensing electrode is formed by using a metal oxide at one side of an oxygen ion conductive solid electrolyte, and a reference electrode is formed by using a precious metal at another side of the oxygen ion conductive solid electrolyte, and an electric potential difference between the sensing electrode and the reference electrode is measured. In other words, the sensing electrode is reactive to a nitrogen-oxide and oxygen, but the reference electrode is only reactive to oxygen, and thus the electric potential difference is generated between the sensing electrode and the reference electrode according to concentration of the nitrogen-oxide gas in a gas. Thus, the concentration of the nitrogen-oxide gas is determined by measuring the electric potential difference. However, measurement accuracy may remarkably decrease in a nitrogen-oxide gas in which a nitrogen dioxide and a nitric oxide are mixed, due to a difference between electromotive force signs generated according to a decomposition reaction of the nitrogen dioxide and the nitric oxide.

Accordingly, a method of using a conversion cell that converts a nitrogen-oxide gas into one gas form is used, but there is a limit to converting the entire nitrogen-oxide gas to a nitric oxide or nitrogen dioxide, and thus it is difficult to measure the entire concentration of the nitrogen-oxide gas.

DESCRIPTION OF THE DRAWINGS

FIG. 1 is a schematic diagram of a nitrogen-oxide gas sensor according to an exemplary embodiment of the present invention;

FIG. 2 is a schematic diagram of a nitrogen-oxide gas sensor according to another exemplary embodiment of the present invention;

FIG. 3 is a schematic diagram of a nitrogen-oxide gas sensor according to another exemplary embodiment of the present invention;

FIG. 4 is a schematic diagram of a nitrogen-oxide gas sensor according to another exemplary embodiment of the present invention;

FIG. 5 is a schematic diagram of a nitrogen-oxide gas sensor according to another exemplary embodiment of the present invention;

FIG. 6 is a schematic diagram of a nitrogen-oxide gas sensor according to another exemplary embodiment of the present invention;

FIG. 7 is a schematic diagram of a nitrogen-oxide gas sensor according to another exemplary embodiment of the present invention;

FIG. 8 is a schematic diagram of a nitrogen-oxide gas sensor according to another exemplary embodiment of the present invention;

FIG. 9 is a schematic diagram of a nitrogen-oxide gas sensor according to another exemplary embodiment of the present invention;

FIG. 10 is a schematic diagram of a nitrogen-oxide gas sensor according to another exemplary embodiment of the present invention;

FIG. 11 is a schematic diagram of a nitrogen-oxide gas sensor according to another exemplary embodiment of the present invention;

FIG. 12 is a schematic diagram of a nitrogen-oxide gas sensor according to another exemplary embodiment of the present invention;

FIG. 13 is a schematic diagram of a nitrogen-oxide gas sensor according to another exemplary embodiment of the present invention;

FIG. 14 is a schematic diagram of a nitrogen-oxide gas sensor according to another exemplary embodiment of the present invention;

FIG. 15 is a graph of a variation of a density of a nitrogen-oxide gas sensor and a variation of a voltage according to a first embodiment of the present invention;

FIG. 16 is a graph of a variation of a voltage with respect to a variation of a density of a nitrogen-oxide gas sensor according to a first embodiment of the present invention;

FIG. 17 is a graph of a variation of a density of a nitrogen-oxide gas sensor and a variation of a voltage according to a second embodiment of the present invention;

FIG. 18 is a graph of a variation of a voltage with respect to a variation of a density of a nitrogen-oxide gas sensor according to a second embodiment of the present invention;

FIG. 19 is a graph of a variation of a density of a nitrogen-oxide gas sensor and a variation of a voltage according to a third embodiment of the present invention; and

FIG. 20 is a graph of a variation of a voltage with respect to a variation of a density of a nitrogen-oxide gas sensor according to a third embodiment of the present invention.

## DETAILED DESCRIPTION OF THE INVENTION

### TECHNICAL PROBLEM

The present invention provides a nitrogen-oxide gas sensor that is able to measure a nitric oxide and a nitrogen dioxide at the same time, ensure sensing accuracy and long stability.

### TECHNICAL SOLUTION

A nitrogen-oxide gas sensor including: an oxide ion conductive solid electrolyte; a first film made of a metal oxide which contacts the solid electrolyte; a second film made of a metal oxide that contacts with the solid electrolyte and is separated from the first film; a power source that applies electric power to the first and second films by electrically connecting a first node to the first film and a second node to the second film; a third film made of a metal oxide that contacts the solid electrolyte, wherein the third film and the first film are connected to the power source in parallel; and a measurement unit that measures the electric potential difference between the first and second nodes.

At least one selected from the group consisting of the first through third films may include a p-type semiconductor metal oxide and an n-type semiconductor metal oxide.

The n-type semiconductor metal oxide may be mixed with the p-type semiconductor metal oxide.

The n-type semiconductor metal oxide may be solid solved with the p-type semiconductor metal oxide.

The film of the first through third films including the n-type semiconductor metal oxide may include a laminate of a film including the p-type semiconductor metal oxide and a buffer film including the n-type semiconductor metal oxide.

The third film may be made of the same metal oxide as the first film.

The nitrogen-oxide gas sensor may further include: a fourth film that contacts with the solid electrolyte and is separated from the third film, wherein the fourth film and the second film are connected to the power source in parallel.

The fourth film may be made of a conductive metal.

The fourth film may be made of a metal oxide.

The fourth film may be made of the same metal oxide as the second film.

The nitrogen-oxide gas sensor may further include: a fifth film that contacts with the solid electrolyte and is made of a metal oxide, wherein the fifth film and the first film are connected to the power source in parallel.

The fifth film may be made of the same metal oxide as the first film.

### ADVANTAGEOUS EFFECTS

According to the present invention described above, the third film and the fifth film are connected to the first film in parallel, and the fourth film is connected to the second film in parallel, thereby increasing measurement accuracy.

Further, the p-type semiconductor metal oxide is used as the metal oxide, and further includes the n-type semiconductor metal oxide, thereby obtaining long stability.

MODE OF THE INVENTION

Hereinafter, the present invention will be described more fully with reference to the accompanying drawings, in which exemplary embodiments of the invention are shown. The invention may, however, be embodied in many different forms and should not be construed as being limited to the embodiments set forth herein; rather, these embodiments are provided so that this disclosure will be thorough and complete, and will fully convey the concept of the invention to those skilled in the art.

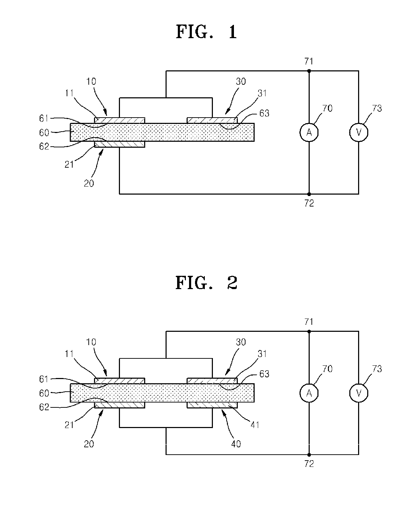

FIG. 1 is a schematic diagram of a nitrogen-oxide gas sensor according to an exemplary embodiment of the present invention.

Referring to FIG. 1, the nitrogen-oxide gas sensor includes an oxygen ion conductive solid electrolyte 60, first and second films 10 and 20 contacting the oxygen ion conductive solid electrolyte 60, a power supply source 70, and a measurement unit 73.

The oxygen ion conductive solid electrolyte 60 is capable of conducting oxygen ions at a high temperature, and may include stabilized zirconia, Ce02, or Th02. Specifically, yttria-stabilized zirconia (YSZ) may be used as the stabilized zirconia.

The first film 10 contacts a first region 61 of the oxygen ion conductive solid electrolyte 60, and the second film 20 contacts a second region 62 of the oxygen ion conductive solid electrolyte 60.

The first and second films 10 and 20 are formed of a first metal oxide electrode 11 and a second metal oxide electrode 12 that are reactive to a nitrogen-oxide and oxygen when power is supplied to the first and second films 10 and 20.

The first metal oxide electrode 11 and the second metal oxide electrode 12 may include a p-type semiconductor metal oxide and may include, for example, at least one material selected from the group, mixtures obtained by mixing at least two materials selected from the group, or a mixtures obtained by mixing the oxygen ion conductive solid electrolyte material and at least one material selected from the group, the group consisting of $CuO$, $NiO$, $CoO$, $Cr_2O_3$, $Cu_2O$, $MoO_2$, $Ag_2O$, $Bi_2O_3$, $Pr_2O_3$, $MnO$, and $LaCoO_3$. In the exemplary embodiment of the present invention, the first metal oxide electrode 11 may include NiO from among the p-type semiconductor metal oxides, and the second metal oxide electrode 12 may include another p-type semiconductor metal oxide that is different from the first film 10, for example, CuO or $LaCoO_3$.

A paste including particles of the metal oxide is coated on the oxygen ion conductive solid electrolyte 60 and is sintered by using a screen printing method and other methods so that the metal oxide can be in a porous shape in the first oxide electrode 11 and the second oxide electrode 21. Therefore, a contact area of the first oxide electrode 11 and the second oxide electrode 21 with the nitrogen-oxide gas may be increased.

Further, the first oxide electrode 11 and the second oxide electrode 21 can be manufactured by including the metal oxide in a paste formed of the same material as the oxygen ion conductive solid electrolyte 60 so that a contact area of the first oxide electrode 11 and the second oxide electrode 21 in the porous shape with the oxygen ion conductive solid electrolyte 60 can be increased.

Referring to FIG. 1, the first and second regions 61 and 62 may face each other in the oxygen ion conductive solid electrolyte 60, but locations of the first and second regions 61 and 62 are not limited thereto, and the first and second regions 61 and 62 may be disposed in different regions on the same plane of the oxygen ion conductive solid electrolyte 60. However, the first and second regions 61 and 62 may not overlap each other. This will be described later in detail.

The first and second films 10 and 20 are electrically respectively connected to a first node 71 and a second node 72 of the power supply source 70, and a uniform current are supplied to the first and second films 10 and 20. In FIG. 1, the first film 10 and the second film 20 are wire bonded to the first node 71 and the second node 72, respectively. That is, a bonding pad is each formed on the first film 10 and the second film 20 by using conductive polymer or other conductive member and a wire power line is connected to the bonding pad.

Meanwhile, the first film 10 and the second film 20 are connected to the power source 70 so that the first film 10 and the second film 20 are used as a positive electrode and a negative electrode, respectively. The power source 70 is not limited to a DC power source and may be an AC power source. Although not shown, an ammeter can be connected to the power source 70 to measure a current supply.

Although not shown, a heater is further installed spaced apart from the oxygen ion conductive solid electrolyte 60 by a predetermined gap to control the oxygen ion conductive solid electrolyte 60 by a temperature for ion conductivity.

An anodic reaction for converting oxygen ions to an oxygen gas is generated on an interface between the first film 10 constituting a positive electrode, and the oxygen ion conductive solid electrolyte 60, and at the same time, if an NO gas exists, the anodic reaction is generated according to the NO gas as shown in Reaction Formula 1 below, and thus a size of a voltage necessary to make a uniform current flow is reduced. In this regard, since anodic polarization is applied to the first film 10, a reaction with respect to NO is big, and a reaction with respect to $NO_2$ is small.

[Reaction Formula 1]

$$O^{2-} = \frac{1}{2}O_2 + 2e^-, NO + O^{2-} = NO_2 + 2e^-$$

A cathodic reaction for converting an oxygen gas to oxygen ions is generated on an interface between the second film 20 constituting a negative electrode, and the oxygen ion conductive solid electrolyte 60, and at the same time, if an $NO_2$ gas exists, the cathodic reaction is generated according to $NO_2$ as shown in Reaction Formula 2 below, and thus a size of a voltage necessary to make a uniform current flow is reduced. In this regard, since cathodic polarization is applied to the second film 20, a reaction with respect to $NO_2$ is big, and a reaction with respect to NO is small.

[Reaction Formula 2]

$$\frac{1}{2}O_2 + 2e^- = O^{2-}, NO_2 + 2e^- \Rightarrow NO + O^{2-}$$

As described above, measurement accuracy of both NO and $NO_2$ may be increased according to the first film 10 and the second film 20 if a nitrogen-oxide gas includes both NO and $NO_2$.

Here, the measurement unit 73 is connected to the first and second nodes 71 and 72 so as to measure an electric potential difference between the first and second nodes 71 and 72.

When the first and second films 10 and 20 are exposed to the nitrogen-oxide gas at a high temperature, an electric potential difference changes according to concentrations of a nitrogen dioxide and a nitric oxide in the nitrogen-oxide gas, and thus a concentration sum of the nitrogen dioxide and the nitric oxide is measured.

Meanwhile, a third film 30 contacts a third region 63 of the oxygen ion conductive solid electrolyte 60.

As illustrated in FIG. 1, the third region 63 may face each other; however, the present invention is not limited thereto. The third region 63 may be formed on the same plane or each different plane as long as the third region 63 is not overlapped with each other. This will be in detail described later.

In this regard, the third film 30 is electrically connected to the first node 71 so that the power source 70 is connected to the first film 10 in parallel.

As such, the first film 10 and the third film 30 are connected in parallel, thereby decreasing a measurement error and increasing long stability. The measurement error is decreased and the long stability is increased by connecting the first and third films 10 and 30 in parallel, thereby dispersing an excess charge generated and/or accumulated on an interface between the first film 10 constituting a measuring electrode, and the oxygen ion conductive solid electrolyte 60 to the third film 30 via an oxygen substitution reaction. However, reasons for the decreased measurement error and increased long stability are not limited thereto, and may be complex.

In the exemplary embodiment of the present invention, the third film 30 is formed of a third oxide electrode 31 contacting the third region 63 of the oxygen ion conductive solid electrolyte 60.

The third oxide electrode 31 may include a p-type semiconductor metal oxide and may include, for example, at least one material selected from the group, mixtures obtained by mixing at least two materials selected from the group, or a mixtures obtained by mixing the oxygen ion conductive solid electrolyte material and at least one material selected from the group, the group consisting of $CuO$, $NiO$, $CoO$, $Cr_2O_3$, $Cu_2O$, $MoO_2$, $Ag_2O$, $Bi_2O_3$, $Pr_2O_3$, $MnO$, and $LaCoO_3$. In the exemplary embodiment of the present invention, the third oxide electrode 31 may be formed of the same material as that of the first oxide electrode 11. The third oxide electrode 31 may be formed of $NiO$. The third oxide electrode 31 may be manufactured in the same manner as the method of manufacturing the first oxide electrode 11 described above.

As described in the embodiment with reference to FIG. 1, the first film 10 and the third film 30 are used as positive electrodes and the second film 20 is used as a negative electrode. In this regard, the first film 10 and the third film 30 comprise the first oxide electrode 11 and the third oxide electrode 31 that react to the nitrogen-oxide gas, respectively, and the second film 20 comprises the second oxide electrode 21 that reacts to the nitrogen-oxide gas, and thus the number of positive electrodes made of the metal oxide is greater than the number of negative electrodes made of the metal oxide, thereby preventing a drift phenomenon and further improving sensing accuracy.

FIG. 2 is a schematic diagram of a nitrogen-oxide gas sensor according to another exemplary embodiment of the present invention. A fourth film 40 contacts a fourth region 64 of the oxygen ion conductive solid electrolyte 60. The fourth film 40 may be electrically connected to the second film 20 in parallel. As such, the second film 20 and the fourth film 40 are connected in parallel, thereby decreasing a measurement error and increasing long stability. The measurement error is decreased and the long stability is increased by connecting the second film 20 and the fourth film 40 in parallel, thereby dispersing an excess charge generated and/or accumulated on an interface between the second film 20 constituting a measuring electrode, and the oxygen ion conductive solid electrolyte 60 to the fourth film 40 via an oxygen substitution reaction. However, reasons for the decreased measurement error and increased long stability are not limited thereto, and may be complex.

The fourth film 40 is formed of a fourth oxide electrode 41 that includes a p-type semiconductor metal oxide and may include, for example, at least one material selected from the group, mixtures obtained by mixing at least two materials selected from the group, or a mixtures obtained by mixing the oxygen ion conductive solid electrolyte material and at least one material selected from the group, the group consisting of $CuO$, $NiO$, $CoO$, $Cr_2O_3$, $Cu_2O$, $MoO_2$, $Ag_2O$, $Bi_2O_3$, $Pr_2O_3$, $MnO$, and $LaCoO_3$. The fourth oxide electrode 41 may be formed of the same material as that of the second oxide electrode 21. The fourth oxide electrode 41 may be formed of $CuO$ or $LaCoO_3$.

The fourth oxide electrode 41 may be manufactured in the same manner as the method of manufacturing the second oxide electrode 21 described above.

Meanwhile, a sum of areas of the fourth oxide electrode 41 and the second oxide electrode 21 may be smaller than a sum of areas of the first oxide electrode 11 and the third oxide electrode 31. Thus, an area of positive electrodes is greater than an area of negative electrodes, thereby preventing the drift phenomenon and further improving sensing accuracy.

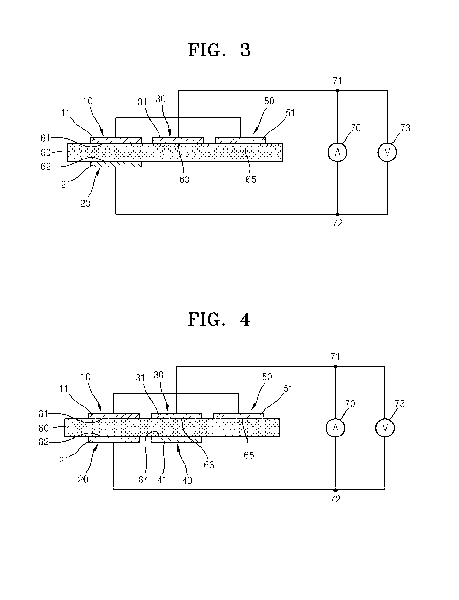

FIG. 3 is a schematic diagram of a nitrogen-oxide gas sensor according to another exemplary embodiment of the present invention. In addition to the exemplary embodiment as described with reference to FIG. 1, a fifth film 50 further contacts a fifth region 65 of the oxygen ion conductive solid electrolyte 60. The fifth film 50 may be electrically connected to the first film 10 and the third film 30 in parallel, thereby decreasing a measurement error and increasing long stability as described above. The fifth film 50 is formed of a fifth oxide electrode 51. The fifth oxide electrode 51 may be formed of the same material as that of the first oxide electrode 11.

FIG. 4 is a schematic diagram of a nitrogen-oxide gas sensor according to another exemplary embodiment of the present invention. In addition to the exemplary embodiment as described with reference to FIG. 2, the fifth film 50 further contacts the fifth region 65 of the oxygen ion conductive solid electrolyte 60. The fifth film 50 may be electrically connected to the first film 10 and the third film 30 in parallel, thereby decreasing a measurement error and increasing long stability as described above. The fifth film 50 is formed of a fifth oxide electrode 51. The fifth oxide electrode 51 may be formed of the same material as that of the first oxide electrode 11.

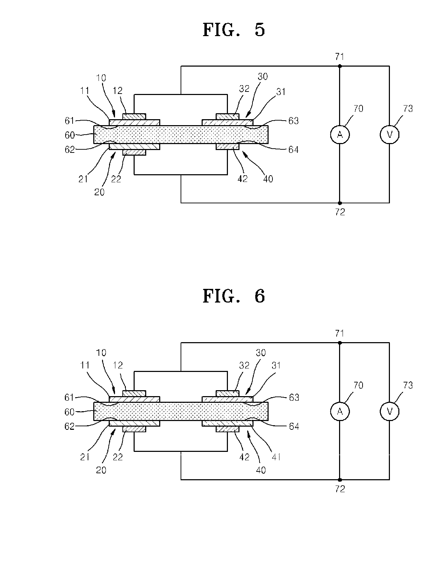

FIG. 5 is a schematic diagram of a nitrogen-oxide gas sensor according to another exemplary embodiment of the present invention.

The first through fourth films 10 through 40 contact the first through fourth regions 61 through 64 of the oxygen ion conductive solid electrolyte 60.

The first through third films 10 through 30 are formed of the first through third metal oxide electrodes 11 through 31 that are reactive to a nitrogen-oxide and oxygen when power is supplied to the first through third films 10 through 30.

In this regard, the first metal electrode 12 can be formed on the first oxide electrode 11, and can be electrically connected to the first node 71. The second metal electrode 22 can be formed on the second oxide electrode 21, and can be electrically connected to the second node 72. The third metal electrode 32 can be formed on the third oxide electrode 31, and can be electrically connected to the first node 71 so that the third metal electrode 32 is connected to the first film 10 in parallel.

The first through third metal electrodes 12 through 32 may be formed of an electric conductive metal, and in detail, may be formed of a precious metal capable of enduring a corrosive environment. At least one selected from the group consisting of gold (Au), silver (Ag), platinum (Pt), iridium (Ir), palladium (Pd), and an alloy thereof may be used as the precious metal, and in detail, gold or platinum may be used as the precious metal.

A paste including particles of the metal is coated on the oxygen ion conductive solid electrolyte 60 or the oxide electrode and is sintered by using a screen printing method and other methods so that the metal oxide can be in a porous shape in the first through third metal electrodes 12 through 32. Therefore, the nitrogen-oxide gas can be penetrated into the first through third metal electrodes 12 through 32.

Such manufacturing of the oxide electrode and the metal electrode can be applied to all oxide electrodes and metal electrodes that will be described later in the same manner.

Meanwhile, the fourth film 40 contacts the fourth region 64 of the oxygen ion conductive solid electrolyte 60.

As illustrated in FIG. 5, the third region 63 and the fourth region 64 may face each other; however, the present invention is not limited thereto. The third region 63 and the fourth region 64 may be formed on the same plane or each different plane as long as the third region 63 and the fourth region 64 are not overlapped with each other.

The fourth film 40 is electrically connected to the second node 72 so that the fourth film 40 and the second film 20 are connected to the power source 70 in parallel. The fourth film 40 includes the fourth metal electrode 42 that contacts the fourth region 64 of the oxygen ion conductive solid electrolyte 60. The fourth metal electrode 42 may be formed of an electric conductive metal, and in detail, may be formed of a precious metal capable of enduring a corrosive environment. At least one selected from the group consisting of gold (Au), silver (Ag), platinum (Pt), iridium (Ir), palladium (Pd), and an alloy thereof may be used as the precious metal, and in detail, gold or platinum may be used as the precious metal. The fourth metal electrode 42 cannot be formed as an electrode in a porous shape and may be formed as a film of a dense structure.

As shown in FIG. 5, each of the third film 30 and the fourth film 40 is not necessarily a single film, and can be formed as a plurality of films. Although not shown, the plurality of third films 30 may be connected to one another in parallel, and the plurality of fourth films 40 may be also connected to one another in parallel.

As described in the embodiment with reference to FIG. 5, the first film 10 and the third film 30 are used as positive electrodes, and the second film 20 and the fourth film 40 are used as negative electrodes, and thus the number of the positive electrodes and the negative electrodes are the same. In this regard, the first film 10 and the third film 30 comprise the first oxide electrode 11 and the third oxide electrode 31 that react to the nitrogen-oxide gas, respectively, and the second film 20 comprises the second oxide electrode 21 that reacts to the nitrogen-oxide gas, and thus the number of positive electrodes made of the metal oxide is greater than the number of negative electrodes made of the metal oxide, thereby preventing the drift phenomenon and further improving sensing accuracy.

Meanwhile, a structure of the fourth film 40 is not necessarily limited to the fourth metal electrode 42 as described above, and may include the fourth oxide electrode 41 and the fourth metal electrode 42 as shown in FIG. 6. The fourth oxide electrode 41 may include a p-type semiconductor metal oxide and may include, for example, at least one material selected from the group, mixtures obtained by mixing at least two materials selected from the group, or a mixtures obtained by mixing the oxygen ion conductive solid electrolyte material and at least one material selected from the group, the group consisting of $CuO$, $NiO$, $CoO$, $Cr_2O_3$, $Cu_2O$, $MoO_2$, $Ag_2O$, $Bi_2O_3$, $Pr_2O_3$, $MnO$, and $LaCoO_3$. The fourth oxide electrode 41 may be formed of the same material as that of the second oxide electrode 21. The fourth oxide electrode 41 may be formed of $CuO$ or $LaCoO_3$. The fourth oxide electrode 41 can be formed as an electrode in a porous shape. In this regard, the fourth metal electrode 42 can be also formed as the electrode in the porous shape so as to facilitate penetrating of the nitrogen-oxide gas into the fourth oxide electrode 41.

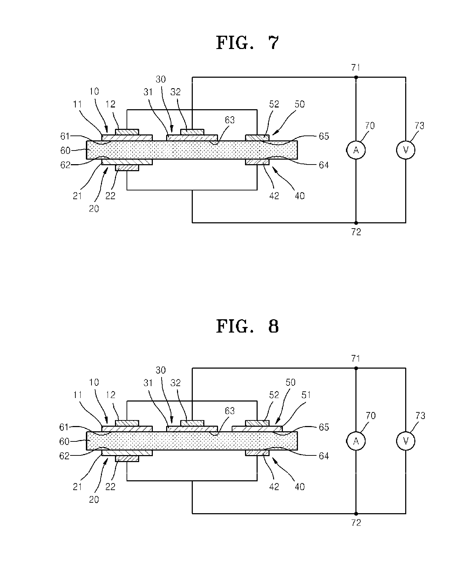

FIG. 7 illustrates a nitrogen-oxide gas sensor according to another exemplary embodiment of the present invention in which the fifth film 50 is further included in the embodiment of FIG. 5. The fifth film 50, the first film 10, and the third film 30 are connected to the power source 70 in parallel so that the fifth film 50 contacts the fifth region 65 of the oxygen ion conductive solid electrolyte 60. Although the fifth film 50 is installed facing the fourth film 40 in FIG. 7, the present embodiment is not limited thereto. The third film 30 may be installed facing the fourth film 40, and the fifth film 50 may be installed without a member facing the fifth film 50.

The fifth film 50 includes the fifth metal electrode 52. The fifth metal electrode 52 may be formed of an electric conductive metal, and in detail, may be formed of a precious metal capable of enduring a corrosive environment. At least one selected from the group consisting of gold (Au), silver (Ag), platinum (Pt), iridium (Ir), palladium (Pd), and an alloy thereof may be used as the precious metal, and in detail, gold or platinum may be used as the precious metal.

In the embodiment of FIG. 7, the number of positive electrodes made of a metal oxide is greater than the number of negative electrodes made of the metal oxide. Further, the number of total electrodes that are positive electrodes is greater than the number of total electrodes that are negative electrodes. If the number of positive electrodes that mainly react to NO is greater than the number of negative electrodes that mainly react to NO2, sensitivities and absolute values to NO and NO2 may be identical to each other, thereby further reducing a measurement error and increasing accuracy of the nitrogen-oxide sensor. In the present invention, the number of the electrodes described above is not limited to the number described in the embodiment and illustrated in the drawings of the present invention. For example, a plurality of films used as positive electrodes, and a plurality of films used as negative electrodes whose number is smaller than the number of the positive electrodes are included in the scope of the present invention.

Meanwhile, as shown in FIG. 8, the fifth film 50 may be installed in such a way that the fifth oxide electrode 51 contacts the oxygen ion conductive solid electrolyte 60, and the fifth metal electrode 52 is formed on the fifth oxide electrode 51.

The fifth oxide electrode 51 may include a p-type semiconductor metal oxide and may include, for example, at least one material selected from the group, mixtures obtained by mixing at least two materials selected from the group, or a mixtures obtained by mixing the oxygen ion conductive solid electrolyte material and at least one material selected from the group, the group consisting of $CuO$, $NiO$, $CoO$, $Cr_2O_3$, $Cu_2O$, $MoO_2$, $Ag_2O$, $Bi_2O_3$, $Pr_2O_3$, $MnO$, and $LaCoO_3$. The fifth oxide electrode 51 may be formed of the same material as that of the second oxide electrode 21. The fifth oxide electrode 51 may be formed of $NiO$. The fifth oxide electrode 51 can be formed as an electrode in a porous shape like the first oxide electrode 11 and the third oxide electrode 31. In this regard, the fifth metal electrode 52 can be also formed as the electrode in the porous shape so as to facilitate penetrating of the nitrogen-oxide gas into the fifth oxide electrode 51.

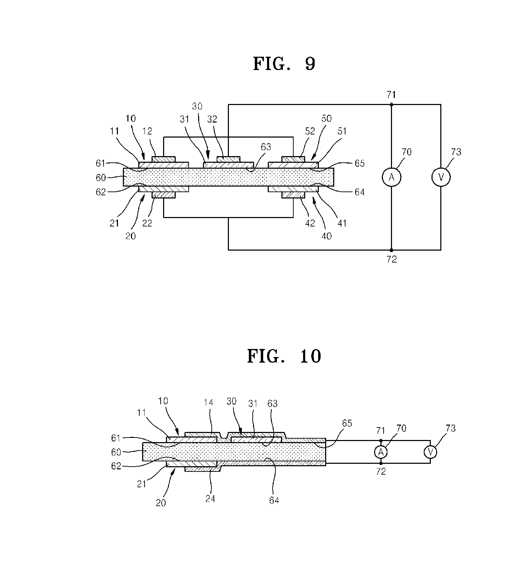

In this regard, the fourth film 40 may only be made of the fourth metal electrode 42. The present embodiment is not necessarily limited thereto. As shown in FIG. 9, the fourth film 40 may be formed having a structure of the fourth oxide electrode 41 and the fourth metal electrode 42. A description of the fourth oxide electrode 41 is the same as the embodiment described with reference to FIG. 6.

Like the embodiment described with reference to FIG. 7, in the embodiments with reference to FIGS. 8 and 9, sensitivities and absolute values to NO and NO2 may be identical to each other in terms of sensitivity and an absolute value, thereby further reducing a measurement error and increasing accuracy of the nitrogen-oxide sensor.

Further, the first film 10 and the second film 20 are connected to the third and fifth films 30 and 50 and the fourth film 40 in parallel, which disperses an excess charge generated and/or accumulated on an interface between the first and second films 10 and 20, and the oxygen ion conductive solid electrolyte 60 to the third and fifth films 30 and 50 and the fourth film 40, thereby further increasing long stability.

Meanwhile, as described above, the nitrogen-oxide sensor uses a -type semiconductor metal oxide as a metal oxide. Thus, the concentration of electrons may be higher than the concentration of holes in the metal oxide, and thus, a gas reaction speed may be gradually decreased due to insufficient electrons, which increases a voltage required for a uniform current according to time. Thus, long stability of the nitrogen-oxide sensor may be reduced. Accordingly, a method of increasing concentration of electrons of a p-type semiconductor metal oxide constituting a sensing material was developed, by manufacturing a solid solution or a mixture of a p-type semiconductor metal oxide that is an oxide electrode and an n-type semiconductor metal oxide or layers thereof. In this regard, the n-type semiconductor metal oxide may be at least one metal oxide selected from the group consisting of ZnO, MgO, Al2O3, Si02, V205, Fe203, SrO, BaO, Ti02, BaTi03, Ce02, Nb205, Ta205, Ga203, and W03. The n-type semiconductor metal oxide may be ZnO.

FIG. 10 is a diagram of a nitrogen-oxide gas sensor according to another embodiment of the present invention. In FIG. 10, as described with reference to FIG. 1, the first through third oxide electrodes 11 through 31 are respectively formed in the oxygen ion conductive solid electrolyte 60, and then, the first and second conductive films 14 and 24 are thinly formed to partially cover the first through third oxide electrodes 11 through 31 on the oxygen ion conductive solid electrolyte 60. The first conductive film 14 is patterned to pass through both first and third oxide electrodes 11 and 31.

The first and second conductive films 14 and 24 may be patterned as a thin film formed of a conductive material, and may operate as wires of the first through third oxide electrodes 11 through 31.

Accordingly, the first and second conductive films 14 and 24 may be formed of a precious metal capable of enduring a corrosive environment, such as at least one metal selected from the group consisting of gold (Au), silver (Ag), platinum (Pt), iridium (Ir), palladium (Pd), and an alloy thereof. In detail, the first and second conductive films 14 and 24 may be patterned by coating with a platinum paste.

In this case, portions covering the first and third oxide electrodes 11 and 31 in the first conductive film 14 may operate as a first metal electrode and a third metal electrode on the first and third oxide electrodes 11 and 31, and a portion contacting the fifth region 65 of the oxygen ion conductive solid electrolyte 60 in the first conductive film 14 may operate as a fifth metal electrode, as shown in FIG. 7. Similarly, a portion covering the second oxide electrode 21 in the second conductive film 24 may operate as a second electrode on the second oxide electrode 21, and a portion contacting the fourth region 64 of the oxygen ion conductive solid electrolyte 60 in the second conductive film 24 may operate as a fourth metal electrode, as shown in FIG. 7.

Meanwhile, in the embodiments described with reference to FIGS. 1 through 10 above, at least one of the first oxide electrode 11 through the fifth oxide electrode 51 is formed of a mixture or a solid solution of the p-type semiconductor metal oxide and the n-type semiconductor metal oxide. The n-type semiconductor metal oxide may be at least one metal oxide selected from the group consisting of ZnO, MgO, Al2O3, Si02, V205, Fe203, SrO, BaO, Ti02, BaTi03, Ce02, Nb205, Ta205, Ga203, and W03, or a mixture thereof, as described above. In this case, long stability of each of the first oxide electrode 11 through the fifth oxide electrode 51 can be obtained.

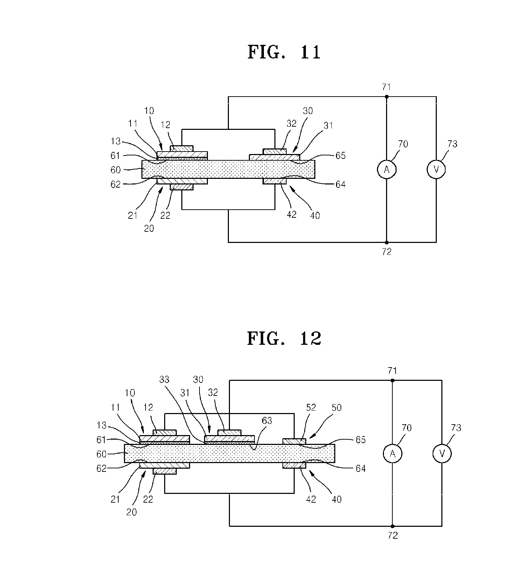

As shown in FIG. 11, the n-type semiconductor metal oxide may be manufactured as a first buffer film 13 disposed between the first oxide electrode 11 and the oxygen ion conductive solid electrolyte 60.

The n-type semiconductor metal oxide included in the first buffer film 13 may include at least one metal oxide selected from the group consisting of ZnO, MgO, Al2O3, SiO2, V2O5, Fe2O3, SrO, BaO, TiO2, BaTiO3, CeO2, Nb2O5, Ta2O5, Ga2O3, and WO3. According to an embodiment of the present invention, the n-type semiconductor metal oxide may be ZnO. Further, the first buffer film 13 is sintered after coating a paste including particles of the n-type semiconductor metal oxide on the oxygen ion conductive solid electrolyte 60 by using a screen printing method so that the first buffer film 13 can be in a porous shape.

A solid solution or a mixture of the p-type semiconductor metal oxide and the n-type semiconductor metal oxide may be used for the first buffer film 13. For example, as shown in FIG. 11, NiO may be used to form the first oxide electrode 11 including the p-type semiconductor metal oxide, and a NiO-ZnO solid solution in which ZnO is employed in NiO may be used to form the first buffer film 13 between the first oxide electrode 11 and the oxygen ion conductive solid electrolyte 60 so as to form the first film 10. In this case, ZnO having a relatively low thermal stability is supplemented, and a mechanical connection characteristic of NiO that is the first oxide electrode 11 with ZnO further increases, thereby further increasing a mechanical connection characteristic of the first film 10 with the oxygen ion conductive solid electrolyte 60.

As such, by disposing the first buffer film 13 between the first oxide electrode 11 including the p-type semiconductor metal oxide, and the oxygen ion conductive solid electrolyte 60, unstable measurement by the nitrogen-oxide gas sensor may be prevented and deterioration of the first film 10 may be delayed, thereby ensuring long stability.

A structure in which a buffer film is interposed like the first buffer film can be applied to all the oxide electrodes described above.

For example, referring to FIG. 12, the first buffer film 13 is interposed between the first oxide electrode 11 of FIG. 7 and the oxygen ion conductive solid electrolyte 60, and a third buffer film 33 is interposed between the third oxide electrode 31 and the oxygen ion conductive solid electrolyte 60. A material of the third buffer film 33 is the same as that of the first buffer film 13.

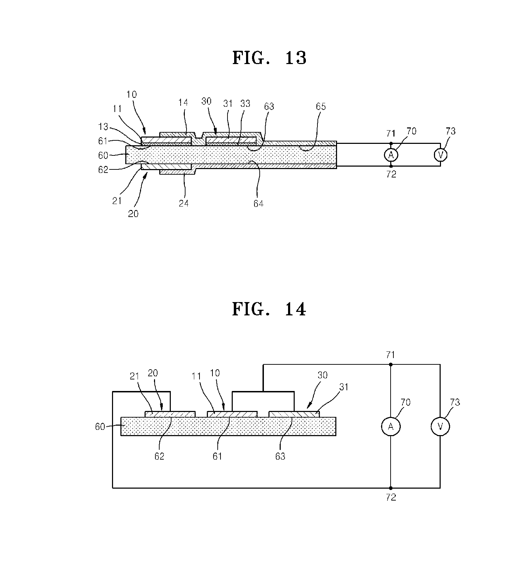

In FIG. 13, the first and second conductive films 14 and 24 are thinly formed as shown in FIG. 10, in stead of the first through fifth metal electrodes 12 through 52 as shown in FIG. 12. That is, as shown in FIG. 12, the first through third oxide electrodes 11 through 31 are respectively formed in the oxygen ion conductive solid electrolyte 60, and then, the first and second conductive films 14 and 24 are thinly formed to partially cover the first through third oxide electrodes 11 through 31 on the oxygen ion conductive solid electrolyte 60. The first conductive film 14 is patterned to pass through both first and third oxide electrodes 11 and 31. The second conductive film 24 is patterned to at least partially cover the second oxide electrode 21.

The first and second conductive films 14 and 24 may be patterned as a thin film formed of a conductive material, and may operate as wires of the first through third oxide electrodes 11 through 31.

Accordingly, the first and second conductive films 14 and 24 may be formed of a precious metal capable of enduring a corrosive environment, such as at least one metal selected from the group consisting of gold (Au), silver (Ag), platinum (Pt), iridium (Ir), palladium (Pd), and an alloy thereof. In detail, the first and second conductive films 14 and 24 may be patterned by coating with a platinum paste.

In this case, portions covering the first and third oxide electrodes 11 and 31 in the first conductive film 14 may operate as a first metal electrode and a third metal electrode on the first and third oxide electrodes 11 and 31, and a portion contacting the fifth region 65 of the oxygen ion conductive solid electrolyte 60 in the first conductive film 14 may operate as a fifth metal electrode, as shown in FIG. 12. Similarly, a portion covering the second oxide electrode 21 in the second conductive film 24 may operate as a second electrode on the second oxide electrode 21, and a portion contacting the fourth region 64 of the oxygen ion conductive solid electrolyte 60 in the second conductive film 24 may operate as a fourth metal electrode, as shown in FIG. 12.

Meanwhile, the structure shown in FIG. 1 may be formed contacting the first region 61 through the third region 63 that are the same planes of the oxygen ion conductive solid electrolyte 60 as shown in FIG. 14. The embodiment of FIG. 14 can be applied to all the embodiments described with reference to FIGS. 2 through 13.

Next, specific embodiments of the present invention as described above will now be explained.

<Comparison Example>

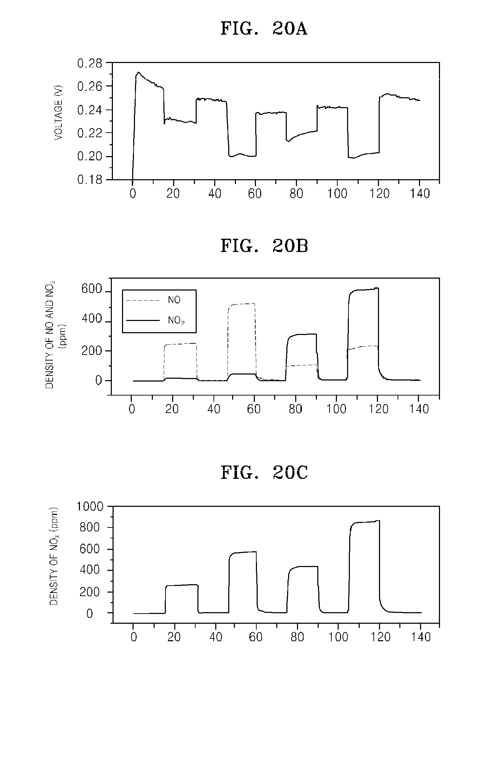

FIG. 20 shows a measurement result when a positive electrode formed of NiO and YSZ and a negative electrode that are formed of CuO are formed on one surface of a stabilized Zirconia solid electrolyte, and a current of 7 pA is applied to the positive electrode and the negative electrode at 700 °C.

Referring to FIG. 20, since changes in density of CO and NO2 and changes in voltage values thereof are similar to each other, it is possible to apply a sensor for measuring a whole amount of a nitrogen-oxide gas. However, a drift phenomenon (a signal is not uniform and continuously rises or falls with respect to time elapsed) occurred in the voltage values when there is no nitrogen-oxide gas, and a sensor had no uniform signal at a uniform density of the nitrogen-oxide gas.

<First Embodiment>

A nitrogen-oxide sensor of a structure as shown in FIG. 5 was manufactured. Yttria Stabilized Zirconia (YSZ) to which oxide yttrium is added was used as the oxygen ion conductive solid electrolyte 60.

The first oxide electrode 11 and the third oxide electrode 31 that are formed of NiO were formed on one surface of the oxygen ion conductive solid electrolyte 60. The first metal electrode 12 and the third metal electrode 32 that are formed of platinum (Pt) were formed on the top portions of the first oxide electrode 11 and the third oxide electrode 31, respectively, were connected in parallel to each other, and were connected to the positive electrode of the power source 70.

The second oxide electrode 21 that is formed of CuO was formed on another surface of the oxygen ion conductive solid electrolyte 60. The second metal electrode 22 that is formed of platinum (Pt) was formed on the top portion of the second oxide electrode 21, and the fourth metal electrode 42 that is formed of platinum (Pt) was formed to contact the fourth region 64 of the oxygen ion conductive solid electrolyte 60. The second metal electrode 22 and the fourth metal electrode 42 were connected in parallel to each other, and were connected to the negative electrode of the power source 70.

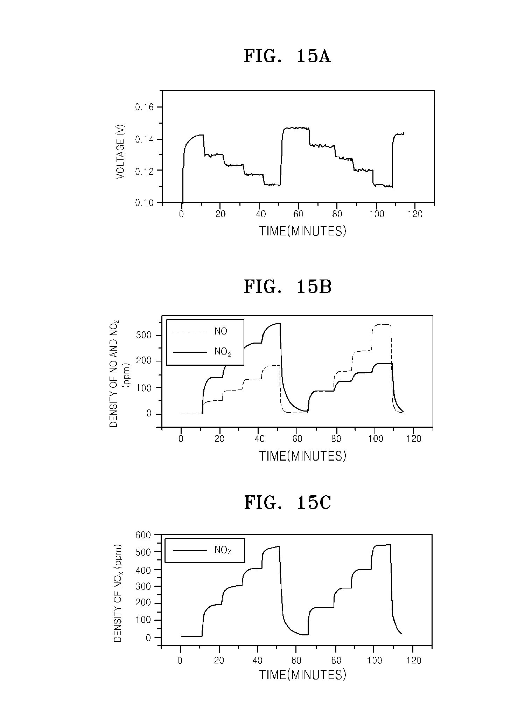

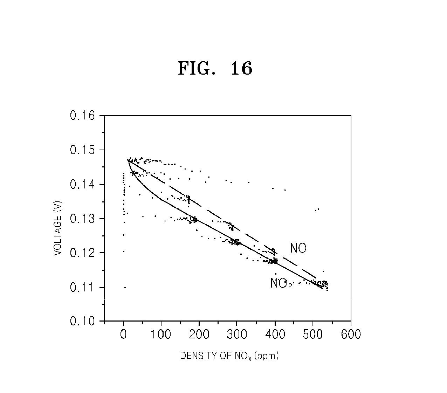

FIG. 15 shows measurements of a variation of a nitrogen-oxide gas and a variation of a voltage when a uniform current of 0.8 pA is applied at 700 °C and a partial pressure of 20% according to the first embodiment. FIG. 16 shows a relationship between the variation of the nitrogen-oxide gas and the variation of a voltage.

Unlike the graph of FIG. 20 according to the conventional art described above, a graph of FIG. 15 shows that the change (a) in the voltage is relatively accurate in correspondence with the change (c) of an entire amount of the nitrogen-oxide gas, no drift phenomenon occurs, and a sensor signal is reliable and accurate. Further, as shown in the graph of FIG. 16, NO and NO2 have an approximately similar tendency in sensitivity that is an inclination of a variation of a voltage value with respect to a variation of a density of a nitrogen-oxide gas, and NO and NO2 are approximately identical to each other in an absolute value that is a section y of the graph.

<Second Embodiment>

A nitrogen-oxide sensor of a structure as shown in FIG. 7 was manufactured. YSZ was used as the oxygen ion conductive solid electrolyte 60.

The first oxide electrode 11 and the third oxide electrode 31 that are formed of NiO were formed on one surface of the oxygen ion conductive solid electrolyte 60. The first metal electrode 12 and the third metal electrode 32 that are formed of platinum (Pt) were formed on the top portions of the first oxide electrode 11 and the third oxide electrode 31, respectively, and the fifth metal electrode 52 that is formed of platinum (Pt) was formed on the fifth region 65 of the oxygen ion conductive solid electrolyte 60. The first metal electrode 12, the third metal electrode 32, and the fifth metal electrode 52 were connected in parallel to one another, and were connected to the positive electrode of the power source 70.

The second oxide electrode 21 that is formed of CuO was formed on another surface of the oxygen ion conductive solid electrolyte 60. The second metal electrode 22 that is formed of platinum (Pt) was formed on the top portion of the second oxide electrode 21, and the fourth metal electrode 42 that is formed of platinum (Pt) was formed to contact the fourth region 64 of the oxygen ion conductive solid electrolyte 60. The second metal electrode 22 and the fourth metal electrode 42 were connected in parallel to each other, and were connected to the negative electrode of the power source 70.

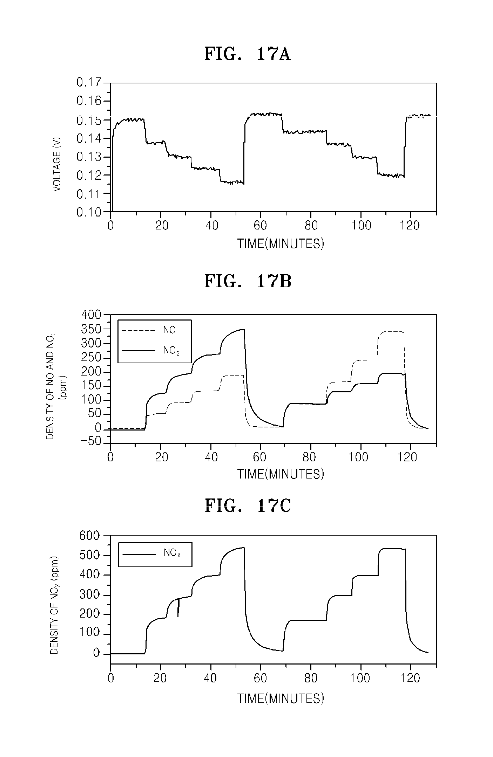

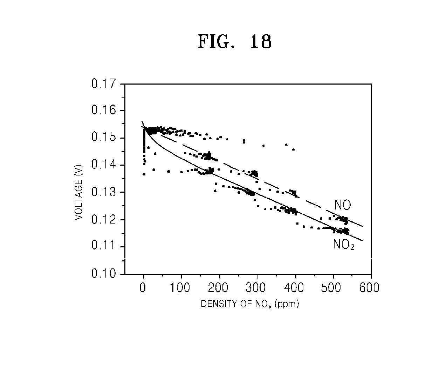

FIG. 17 shows measurements of a variation of a nitrogen-oxide gas and a variation of a voltage when a uniform current of 0.9 pA is applied at 700 °C and a partial pressure of 20% according to the second embodiment. FIG. 18 shows a relationship between the variation of the nitrogen-oxide gas and the variation of a voltage.

A graph of FIG. 17 shows that the change (a) in the voltage is relatively accurate in correspondence with the change (c) of an entire amount of the nitrogen-oxide gas, no drift phenomenon occurs, and a sensor signal is reliable and accurate. Further, as shown in the graph of FIG. 18, NO and NO2 are approximately identical to each other in terms of sensitivity and an absolute value.

<Third Embodiment>

A nitrogen-oxide sensor of a structure as shown in FIG. 12 was manufactured. YSZ was used as the oxygen ion conductive solid electrolyte 60.

The first buffer film 13 and the third buffer film 33 that are solid solutions of NiO and ZnO were formed on one surface of the oxygen ion conductive solid electrolyte 60. The first oxide electrode 11 and the third oxide electrode 31 were formed on the first buffer film 13 and the third buffer film 33. The first metal electrode 12 and the third metal electrode 32 that are formed of platinum (Pt) were formed on the top portions of the first oxide electrode 11 and the third oxide electrode 31, respectively, and the fifth metal electrode 52 that is formed of platinum (Pt) was formed on the fifth region 65 of the oxygen ion conductive solid electrolyte 60. The first metal electrode 12, the third metal electrode 32, and the fifth metal electrode 52 were connected in parallel to one another, and were connected to the positive electrode of the power source 70.

The second oxide electrode 21 that is formed of CuO was formed on another surface of the oxygen ion conductive solid electrolyte 60. The second metal electrode 22 that is formed of platinum (Pt) was formed on the top portion of the second oxide electrode 21, and the fourth metal electrode 42 that is formed of platinum (Pt) was formed to contact the fourth region 64 of the oxygen ion conductive solid electrolyte 60. The second metal electrode 22 and the fourth metal electrode 42 were connected in parallel to each other, and were connected to the negative electrode of the power source 70.

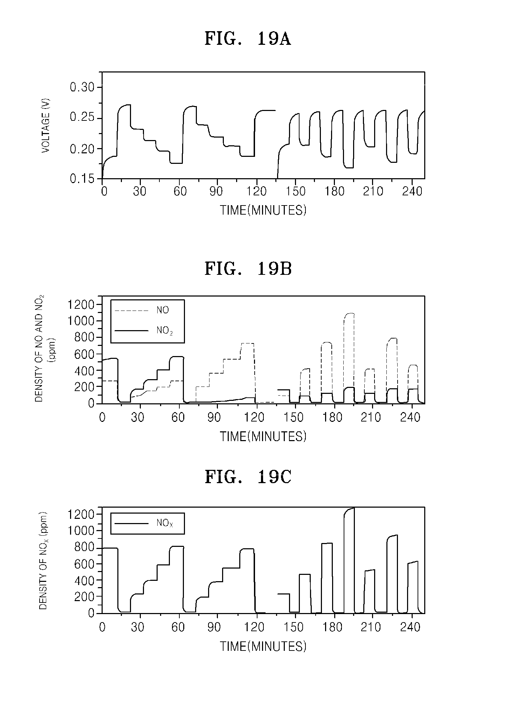

FIG. 19 shows measurements of a variation of a nitrogen-oxide gas and a variation of a voltage when a uniform current of 2.5 pA is applied at 700 °C and a partial pressure of 20% according to the third embodiment.

A graph of FIG. 19 shows that the change (a) in the voltage is relatively accurate in correspondence with the change (c) of an entire amount of the nitrogen-oxide gas, no drift phenomenon occurs, and a sensor signal is reliable and accurate. Although sensing is continued for a long time, a voltage value maintains constant when a density of the nitrogen-oxide gas is zero, and a variation of the voltage value corresponding to a variation of a density of NOx is relatively accurate, and thus long stability is excellent.

As described above, the present invention can be used as a nitrogen-oxide gas sensor and a nitrogen-oxide processing device for home, vehicle, and industry.

**Claims**

1. A nitrogen-oxide gas sensor comprising:

   an oxide ion conductive solid electrolyte;
   a first film made of a metal oxide which contacts the solid electrolyte;
   a second film made of a metal oxide that contacts with the solid electrolyte and is separated from the first film;
   a power source that applies electric power to the first and second films by electrically connecting a first node to the first film and a second node to the second film;
   a third film made of a metal oxide that contacts the solid electrolyte, wherein the third film and the first film are connected to the power source in parallel; and
   a measurement unit that measures the electric potential difference between the first and second nodes.

2. The nitrogen-oxide gas sensor of claim 1, wherein at least one selected from the group consisting of the first through third films comprises a p-type semiconductor metal oxide and an n-type semiconductor metal oxide.

3. The nitrogen-oxide gas sensor of claim 2, wherein the n-type semiconductor metal oxide is mixed with the p-type semiconductor metal oxide.

4. The nitrogen-oxide gas sensor of claim 2, wherein the n-type semiconductor metal oxide is solid solved with the p-type semiconductor metal oxide.

5. The nitrogen-oxide gas sensor of claim 2, wherein the film of the first through third films comprising the n-type semiconductor metal oxide comprises a laminate of a film comprising the p-type semiconductor metal oxide and a buffer film comprising the n-type semiconductor metal oxide.

6. The nitrogen-oxide gas sensor of any one of claims 1 through 5, wherein the third film is made of the same metal oxide as the first film.

7. The nitrogen-oxide gas sensor of any one of claims 1 through 5, further comprising: a fourth film that contacts with the solid electrolyte and is separated from the third film, wherein the fourth film and the second film are connected to the power source in parallel.

8. The nitrogen-oxide gas sensor of claim 7, wherein the fourth film is made of a conductive metal.

9. The nitrogen-oxide gas sensor of claim 7, wherein the fourth film is made of a metal oxide.

10. The nitrogen-oxide gas sensor of claim 10, wherein the fourth film is made of the same metal oxide as the second film.

11. The nitrogen-oxide gas sensor of any one of claims 1 through 5, further comprising: a fifth film that contacts with the solid electrolyte and is made of a metal oxide, wherein the fifth film and the first film are connected to the power source in parallel.

12. The nitrogen-oxide gas sensor of claim 11, wherein the fifth film is made of the same metal oxide as the first film.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15A

FIG. 15B

FIG. 15C

FIG. 16

FIG. 17A

FIG. 17B

FIG. 17C

FIG. 18

## FIG. 19A

## FIG. 19B

## FIG. 19C

## FIG. 20A

## FIG. 20B

## FIG. 20C